(19) Europäisches Patentamt
European Patent Office
Office européen des brevets

(11) Veröffentlichungsnummer: **0 369 288 A1**

(12) # EUROPÄISCHE PATENTANMELDUNG

(21) Anmeldenummer: **89120560.1**

(22) Anmeldetag: **07.11.89**

(51) Int. Cl.$^5$: **C07D 309/30, C07C 69/732, C07C 53/21**

(30) Priorität: **14.11.88 US 270980**

(43) Veröffentlichungstag der Anmeldung: **23.05.90 Patentblatt 90/21**

(84) Benannte Vertragsstaaten: **AT BE CH DE FR GB IT LI LU NL SE**

(71) Anmelder: **F. HOFFMANN-LA ROCHE AG**
**Postfach 3255**
**CH-4002 Basel(CH)**

(72) Erfinder: **Goldstein, Ann**
**90 Clarewill Avenue**
**Upper Montclair, N.J. 07043(US)**
Erfinder: **McLane, John Arthur**
**24 Howard Street**
**West Haven Connecticut 06516(US)**
Erfinder: **Uskokovic, Milan Radoje**
**253 Highland Avenue**
**Upper Montclair, N.J. 07043(US)**
Erfinder: **Wovkulich, Peter Michael**
**124 Rhoda Avenue**
**Nutley, N.J. 07110(US)**

(74) Vertreter: **Grossner, Lutz, Dr. et al**
**Grenzacher Strasse 124 Postfach 3255**
**CH-4002 Basel(CH)**

(54) **Pyranyläthyl-naphathalinderivate, ihre Herstellung und Verwendung als Heilmittel.**

(57) Die verbindungen der Formeln

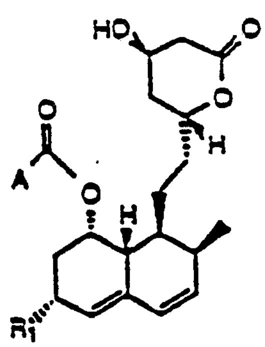

I

und

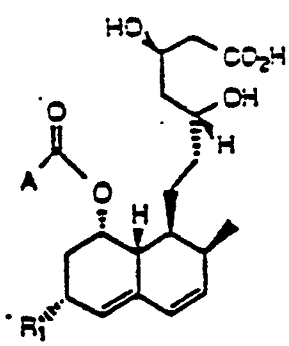

II

worin A und $R_1$ die in der Beschreibung angegebenen Bedeutung haben, sowie Salze, $C_{1-4}$-Alkylester, Acetylamino-$C_{1-4}$-alkylester, Phenyl-$C_{1-4}$-alkylester, Dimethylamino-$C_{1-4}$-alkylester und α-Monoglyceride der Verbindungen der Formel II können als Heilmittel zur Behandlung hyperproliferativer Erkrankungen, wie Psoriasis, und Hypercholesterinämie verwendet werden.

EP 0 369 288 A1

Xerox Copy Centre

## Pyranyläthyl-naphthalinderivate, ihre Herstellung und Verwendung als Heilmittel

Die Erfindung betrifft Verbindungen der Formel

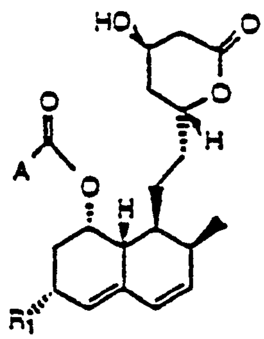
I

und

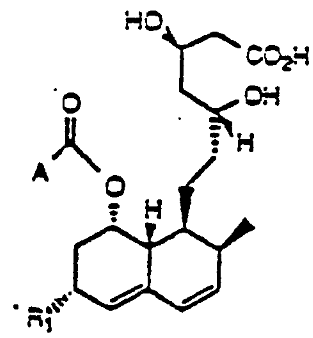
II

worin $R_1$ Wasserstoff oder Methyl, A $R_2$-$C(F)_2$-$C(CH_3)_2$- oder $R_3O$-$C(CH_3)(R_4)$-ist, wobei $R_2$ Niederalkyl, $R_3$ Phenyl, oder Phenyl, das durch 1-3 Substituenten aus der Gruppe Halogen, Niederalkyl und Niederalkoxy substituiert ist, und $R_4$ Wasserstoff oder Methyl darstellt,
und pharmazeutisch anwendbare Salze, $C_{1-4}$-Alkylester, Acetylamino-$C_{1-4}$-alkylester, Phenyl-$C_{1-4}$-alkylester, Dimethylamino-$C_{1-4}$-alkylester und $\alpha$-Monoglyceride der Verbindungen der Formel II.

Die Verbindungen der Formel I und II und die oben bezeichneten Salze, Ester und Monoglyceride der Verbindungn der Formel II sind bei der Behandlung von hyperproliferativen Hauterkrankungen wie Psoriasis, Basalzellkarzinomen, Plattenepithelkarzinomen, Keratosis und Keratinisierungsstörungen von Wert. Die Verbindungen können entweder oral, oder topisch auf die psoriatische Haut verabreicht werden.

Die Verbindungen der Formeln I und II und die oben bezeichneten Salze, Ester und Monoglyceride von Verbindungen der Formel II sind wirksam als Antagonisten der Hauthyperproliferation, d.h. als Mittel, die die Hyperproliferation menschlicher Keratinozyten hemmen. Die Verbindungen antagonisieren ferner Veränderungen in der Differenzierung von Keratinozyten. Die Verbindungen sind infolgedessen als Mittel zur Behandlung hyperproliferativer Hauterkrankungen wie Psoriasis, Basalzellkarzinomen, Keratinisierungsstörungen und Keratosis von Wert.

Der hier verwendete Ausdruck "Psoriasis" bezeichnet eine hyperproliferative Hauterkrankung, die den Regulierungsmechanismus der Haut verändert. Insbesondere werden Läsionen gebildet, die primäre und sekundäre Veränderungen der Proliferation in der Epidermis, entzündliche Reaktionen der Haut und die Expression regulatorischer Moleküle wie Lymphokine und Entzündungsfaktoren beinhalten. Psoriatische Haut ist morphologisch durch einen verstärkten Umsatz von Epidermiszellen, vereickte Epidermis, abnormale Keratinisierung entzündlicher Zellinfiltrate in die Dermisschicht und polymorphonucleäre Leukozyteninfiltration in die Epidermis, die eine Zunahme des Basalzellzyklus bedingt, gekennzeichnet. Zusätzlich sind hyperkeratotische und parakeratotische Zellen anwesend.

Der Ausdruck "Keratosis", "Basalzellkarzinome", "Plattenepithelzellkarzinome" und "Keratinisierungsstörungen" bezieht sich auf hyperproliferative Hauterkrankungen bei denen den Regulierungsmechanismus für die Proliferation und Differenzierung der Hautzellen unterbrochen ist.

Der Ausdruck "Phenyl das durch 1-3 Substituenten" substituiert ist bezeichnet einen Phenylrest in dem ein oder mehrere Wasserstoffe durch einen Substituenten ersetzt sind.

Der Ausdruck "$C_{1-4}$-Alkyl" bezeichnet eine Alkylgruppe mit 1-4 C-Atomen.

In den hier dargestellten Formeln bezeichnet eine keilförmig verdickte Linie einen Substituenten, der oberhalb der Ebene des Moleküls liegt ($\beta$-Stellung) eine unterbrochene Linie (ııııı) oder (---) einen Substituenten der unterhalb der Molekülebene liegt ($\alpha$-Stellung).

Der hier verwendete Ausdruck "Niederalkyl" bezeichnet allein oder in Kombination einen geradkettigen oder verzweigtkettigen gesättigten Kohlenwasserstoffrest, der vorzugsweise 1-6 C-Atome enthält, beispielsweise Methyl, Aethyl, Propyl, Isopropyl, Butyl, tert.-Butyl, Pentyl und Hexyl. Der Ausdruck "Niederalkoxy" bezeichnet. einen Alkoxyrest mit 1-6 C-Atomen wie Methoxy, Aethoxy, Propoxy. Der Ausdruck "Halogen" bezeichnet Brom, Jod, Chlor und Fluor.

Die Erfindung betrifft auch Verfahren zur Herstellung der Verbindung der Formel I und II und der oben bezeichneten Salze, Ester und Monoglyceride von Verbindungen der Formel II und die Verwendung der Verbindungen der Formeln I oder II oder eines der oben bezeichneten Salze, Ester oder Monoglyceride zur

Herstellung von Medikamenten für die orale oder topische Verabreichung bei der Behandlung hyperproliferativer Hauterkrankungen wie Psoriasis.

Erfindungsgemäss können die Verbindungen der Formel I wie in Reaktionsschema I dargestellt, hergestellt werden.

Reaktionsschema I

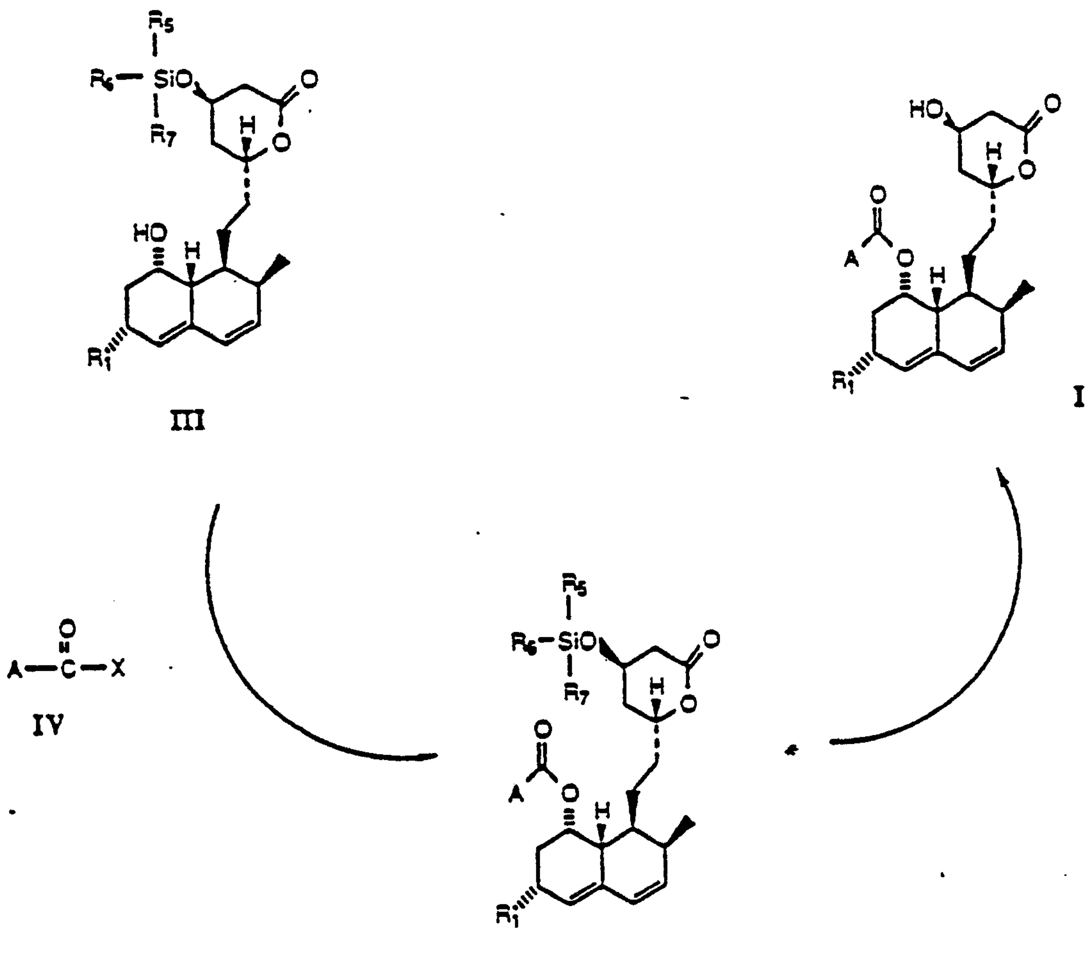

III

I

IV

I'

A und $R_1$ wie oben, X Chlor oder Brom, $R_5$, $R_6$ und $R_7$ unabhängig Niederalkyl oder Phenyl, vorausgesetzt, dass nicht mehr als zwei der Reste $R_5$, $R_6$ und $R_7$ Phenyl sind.

Die Verbindungen der Formel III im Reaktionsschema I sind bekannt oder können nach an sich bekannten Methoden hergestellt werden, beispielsweise wie im U.S. Patent 4,444,784 beschrieben.

Verbindungen der Formel IV worin A $R_2$-C(F)$_2$-C($CH_3$)$_2$-; X Chlor oder Brom und $R_2$ Niederalkyl ist, sind neu und können wie weiter unten beschrieben, hergestellt werden. Die Verbindung der Formel IV wird mit einer Verbindung der Formel III in Gegenwart einer Base wie Triäthylamin, Imidazol oder, insbesondere, Pyridin, 4-N,N-Dimethylaminopyridin oder 4-Pyrrolidinopyridin, gewünschtenfalls in Gegenwart eines organischen Lösungsmittels wie Tetrahydrofuran, Dichlormethan oder Acetonitril bei etwa Raumtemperatur bis etwa Rückflusstemperatur des Lösungsmittels, vorzugsweis bei etwa 50°C umgesetzt, wobei man nach konventioneller Aufarbeitung eine Verbinung der Formel I′ erhält.

Die Verbindung der Formel I′ wird mit einem Reagens umgesetzt, das die Silyläthergruppe spaltet, beispielsweise verdünnte, wässrige Fluorwasserstoffsäure oder, vorzugsweise, wässriges Tetrabutylammoniumfluorid, das mit einer Säure wie Trifluoressigsäure, Essigsäure oder einer Mineralsäure wie Salzsäure gepuffert wird, in einem Lösungsmittel wie Methanol, Aethanol oder Dioxan oder, insbesondere, Tetrahydrofuran. Nach konventioneller Aufarbeitung, z.B. Extraktion mit einem organischen Lösungsmittel wie trockenem Diäthyläther erhält man eine Verbindung der Formel I.

Die Verbindungen der Formel I können mit Basen wie Natronlauge hydrolysiert werden und liefern die entsprechenden Salze, z.B. die Natriumsalze. Sorgfältige Ansäuerung der Salze liefert die entsprechenden Hydroxysäuren der Formel II. Die Verbindungen der Formel II können umgekehrt in Verbindungen der Formel I bei saurem pH umgewandelt werden.

Weiterhin können die Verbindungen der Formel I unter saurer oder basischer Katalyse mit Methanol, Aethanol, Propanol oder Butanol oder mit Phenyl-, Dimethylamino-, oder Acetylaminoalkanolen zu den entsprechenden Estern der Hydroxysäuren der Verbindungen der Formel II umgesetzt werden.

Bevorzugte Verbindungen der Formel I sind:

2-(4-Chlorphenoxy-2-methylpropionsäure [1S-[1α,3α,7β,8β(2R*,4R*),8aβ]]-1,2,3,7,8,8a-hexahydro-3,7-dimethyl-8-[2-[tetrahydro-4-hydroxy-6-oxo-2H-pyran-2-yl]äthyl]-1-naphthalinylester und

3,3-Difluor-2,2-dimethylbutansäure, [1S-[1α,3α,7β,8β(2R*,4R*)8aβ]]-8-[2[tetrahydro-4-hydroxy-6-oxo-2H-pyran-2-yl]äthyl]-1,2,3,7,8,8a-hexahydro-3,7-dimethyl-1-naphthalinylester.

Die Verbindungen der Formel IV, in denen A $R_3O-C(CH_3)(R_4)$- ist, sind bekannt oder können nach an sich bekannten Methoden hergestellt werden, wie Umsetzung der entsprechenden Carbonsäuren mit einem ein Säurehalogenid bildenden Reagens wie Thionylchlorid, Thionylbromid oder Oxalylchlorid in einem konventionellen Lösungsmittel für derartige Reaktionen, wie Dichlormethan.

Verbindungen der Formel IV, in denen A $R_2-C(F)_2-C(CH_3)_2$- ist, sind neu und ebenfalls Gegenstand der Erfindung, wie auch die entsprechenden Carbonsäuren der Formel

$R_2-C(F)_2-C(CH_3)_2-COOH$ IV′

Die Verbindungen der Formel IV′ können wie im Reaktionsschema II dargestellt, erhalten werden.

## Reaktionsschema II

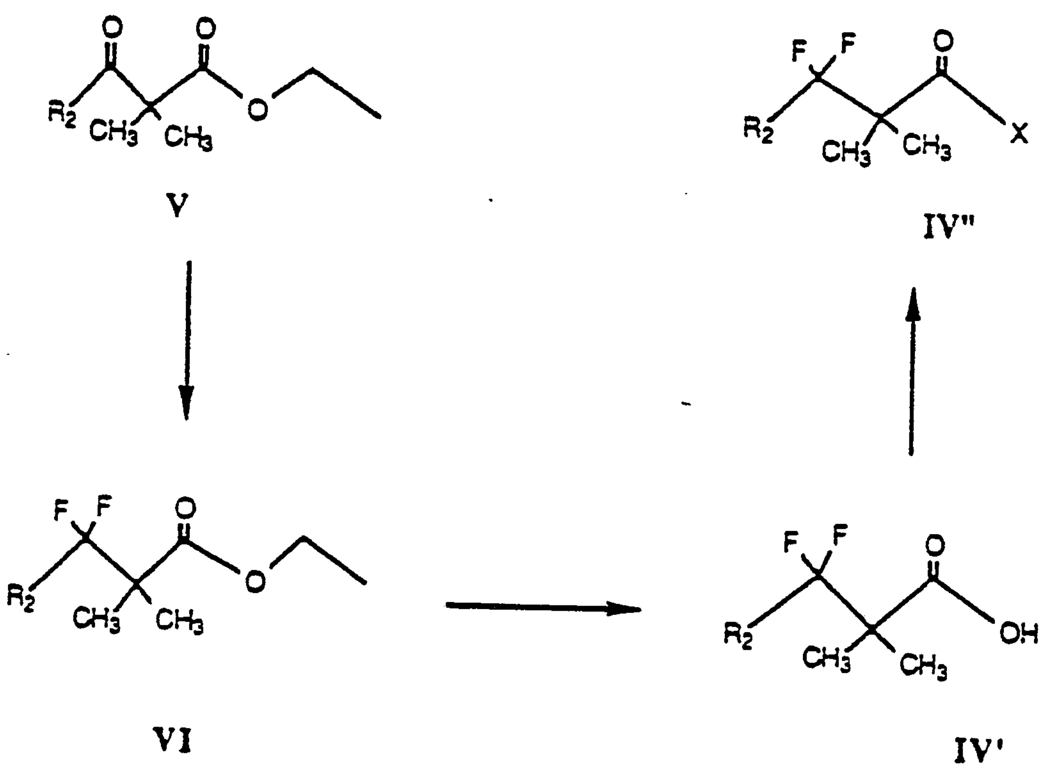

V

IV"

VI

IV'

Verbindungen der Formel V sind bekannt oder können in an sich bekannter Weise hergestellt werden.

Eine Verbindung der Formel V wird mit einem selektiven Fluorierungsmittel wie Schwefeltetrafluorid oder Diäthylaminoschwefeltrifluorid zu einer Verbinung der Formel VI umgesetzt.

Eine Verbindung der Formel VI wird mit einem Esterspaltungsmittel wie wässriger Mineralsäure, z.B. Salzsäure oder Schwefelsäure oder einer wässrigen Base wie Lithium oder Kaliumhydroxid oder, insbesondere, wässrigem Natriumhydroxid in einem organischen Lösungsmittel wie Tetrahydro furan oder Aethanol, oder vorzugsweise ohne ein solches Lösungsmittel, umgesetzt, worauf man nach konventioneller Aufarbeitung eine Verbindung der Formel IV′ erhält.

Eine Verbindung der Formel IV′ wird in eine Verbindung der Formel IV″ umgewandelt durch Behandlung mit einem ein Säurehalogenid bildenden Mittel, wie oben beschrieben.

Beispiele von Verbindungen der Formel IV worin A $R_2C(F)_2$-$C(CH_3)_2$- ist, sind
3,3-Difluor-2,2-dimethylbutansäure;
3,3-Difluor,-2,2-dimethylpentansäure und
3,3-Difluor-2,2-dimethylhexansäure.

Die Verbindungen der Formel I und II und die oben bezeichneten Salze, Ester und Monoglyceride der Verbindungen der Formel II können, wie oben beschrieben, oral verabreicht werden zur Behandlung hyperproliferativer Hauterkrankungen wie Psoriasis, Basalzellkarzinomen, Keratinisierungsstörungen und Keratosis an Warmblüter, die einer solchen Behandlung bedürfen. Insbesondere können die Verbindungen der Formel I wie oben beschrieben, oral an Erwachsenen in Dosierungen im Bereich von etwa 10 bis etwa 80 mg pro Tag zur Behandlung hyperproliferativer Hauterkrankungen, wie Psoriasis, Basalzellkarzinomen, Plattenepithelzellkarzinomen, Keratinisierungsstörungen und Keratosis verabreicht werden.

Die Verbindungen der Formel I und II können auch topisch wie oben beschrieben angewandt werden. Insbesondere können die Verbindungen topisch in Dosierungen die etwa 100-200 Microgramm pro Gramm Formulierung erhalten pro Tag bei solchen Erkrankungen verabreicht werden, insbesonere in einer Menge von 1-50 Microgramm pro Gramm topische Formulierung pro Tag.

Die Wirksamkeit der erfindungsgemässen Verbindungen als Mittel zur Behandlung hypoproliferativer Hauterkrankungen lässt sich durch die Fähigkeit der Verbindungen zeigen, die Proliferation von Keratinozyten in Zellkulturen von Keratinozyten aus Vorhäuten Neugeborener zu hemmen. Die Resultate sind in Tabelle I dargestellt.

TABELLE I

| HEMMWIRKUNG DER TESTVERBINDUNGEN AUF DIE KERATINOZYTENPROLIFERATION | | | |
|---|---|---|---|
| Verbindung | Dosierung der Verbind. (M) | %-Hemmung der Keratinocyten-Proliferation | Standardabweichung |
| 1. Aethanol (Kontrolle) | | 0.00 | 24.48 |
| 2. A | $10^{-10}$ | 20.92 | 40.81 |
| | $10^{-8}$ | 6.28 | 24.83 |
| | $10^{-7}$ | 15.23 | 27.09 |
| | $10^{-6}$ | 49.71 | 23.69 |
| 3. B | $10^{-10}$ | 17.97 | 24.57 |
| | $10^{-8}$ | 14.61 | 24.12 |
| | $10^{-7}$ | 31.23 | 23.81 |
| | $10^{-6}$ | 95.80 | 29.03 |

In der obigen Tabelle ist A
2-(4-Chlorophenoxy-2-methylpropionsäure [1S-[1α,3α,7β,8β(2R*,4R*),8aβ]]-1,2,3,7,8,8a-hexahydro-3,7-dimethyl-8-[2-[tetrahydro-4-hydroxy-6-oxo-2H-pyran-2-yl]äthyl]-1-naphthalinylester; und
3,3-Difluor-2,2-dimethylbutansäure [1S-[1α,3α,7β,8β(2R*,4R*)8aβ]]-8-[2[tetrahydro-4-hydroxy-6-oxo-2H-pyran-2-yl]äthyl]-1,2,3,7,8,8a-hexahydro-3,7-dimethyl-1-naphthalinylester.

Erfindungsgemäss wurde weiterhin gefunden, dass die Verbindungen der Formel I und II und die oben bezeichneten Salze, Ester und Monoglyceride der Verbindungen der Formel II eine cholesterinspiegelsenkende Aktivität aufweisen.

Orale Dosierungsformen mit den erfindungsgemässen Verbindungen können Kapseln, Tabletten oder ähnliches sein.

Topische Formulierungen umfassen Gele, Crèmes, Lotionen, Salben, Puder, Aerosole und andere herkömmliche Formulierungen zur Anwendung von Heilmitteln auf die Haut. Neben der Anwendung auf die Haut können die topischen Formulierungen der vorliegenden Erfindung auch angewandt werden bei der

Behandlung von Entzündung von Schleimhäuten die der topischen Behandlung zugänglich sind. Beispielsweise können die topischen Formulierungen auf die Schleimhäute des Mundes oder des unteren Colons aufgebracht werden.

Die nachstehenden Beispiele erläutern die Erfindung weiter. Die Temperaturen sind in Celsius Graden angegeben.

## Beispiel 1

Ein Gemisch von 1 g [1S-[1α(4R*,6R*),2α,6β,8β,8aα]]-4-[[(1,1-Dimethyläthyl)dimethylsilyl]oxy]-6-[2-(1,2,6,7,8,8α-hexahydro-8-hydroxy-2,6-dimethyl-1-naphthalinyl)äthyl]tetrahydro-2H-pyran-2-on in 18,6 ml trockenem Pyridin, 1,4 g 4-N,N-Dimethylaminopyridin und 5,34 g 2-(4-Chlorphenoxy)-2-methylpropionylchlorid wurde 6 3/4 Stunden auf 50° erwärmt, dann über Nacht bei Raumtemperatur gerührt. Danach wurden Eisstücke zugegeben, das Gemisch nach 30 Minuten in Eiswasser gegossen und viermal mit Diäthyläther extrahiert. Die vereinigten ätherischen Extrakte wurden mit Wasser und dann mit 0,1N Schwefelsäure gewaschen und getrocknet. Das Gemisch wurde abfiltriert und die flüchtigen Bestandteile unter vermindertem Druck entfernt. Der Rückstand wurde in Diäthyläther gelöst, die Lösung mit gesättigter Natriumbicarbonatlösung gewaschen und über Natriumsulfat getrocknet. Das Gemisch wurde abfiltriert, die flüchtigen Stoffe unter vermindertem Druck ent fernt, worauf man 1,6 g 2-(4-Chlorphenoxy)-2-methylpropionsäure-[1S-[1α,3α,7β,8β(2R*,4R*),8aβ]]-8 [2[tetrahydro-4-[[(1,1-dimethyläthyl)dimethylsilyl]oxy]-6-oxo-2H-pyran-2- yl]äthyl]-1,2,3,7,8,8a-hexahydro-3,7-dimethyl-1-naphthalinylester als braunes Oel erhielt, das ohne weitere Reinigung verwendet wurde.

Das so erhaltene Rohprodukt wurde mit 5,6 ml Tetrahydrofuran, 0,29l ml Wasser, 0,402 ml Essigsäure und 5,22 ml 1M Tetrabutylammoniumfluorid in Tetrahydrofuran 23 Stunden gerührt. Das Gemisch wurde in Diäthyläther aufgenommen, mit Kochsalzlösung gewaschen, über Natriumsulfat getrocknet, filtriert und eingedampft. Der Rückstand wurde über Silicagel filtriert und lieferte nach Eindampfen 0,91 g 2-(4-Chlorphenoxy)-2-methylpropionsäure [1S-[1α,3α,7β,8β(2R*,4R*),8aβ]]-1,2,3,7,8,8a-hexahydro-3,7-dimethyl-8-[2-[tetrahydro-4-hydroxy-6-oxo-2H-pyran-2-yl]äthyl]-1-naphthalinylester. Diese Verbindung wurde als Silicagel mit Hexan/Aethylacetat (15:85) chromatographiert und lieferte 0,376 g 2-(4-Chlorphenoxy)-2-methylpropionsäure [1S-[1α,3α,7β,8β(2R*,4R*),8aβ]]-1,2,3,7,8,8a-hexahydro-3,7-dimethyl-8-[2-[tetrahydro-4-hydroxy-6-oxo-2H-pyran-2-yl]äthyl]-1 naphthalinylester, das nach Ausfällen aus Hexan/1-Chlorbutan einen amorphen Feststoff mit einem Schmelzpunkt von 65° lieferte, [$\alpha^{25}$ = +226,1 ($CHCl_3$, C, 0,52).

## Beispiel 2

Zu 1,55 g 3,3-Difluor-2,2-dimethylbutansäure in 5 ml trockenem Dichlormethan wurden 3,45 ml Oxalylchlorid gegeben. Das Gemisch wurde 20 Stunden bei Raumtemperatur gerührt, danach wurde das Lösungsmittel und überschüssiges Reagens abdestilliert, worauf Tetrachlorkohlenstoff zugesetzt wurde und das Lösungsmittel erneut abdestilliert wurde. Das gekühlte 3,3-Difluor-2,2-dimethylbutansäurechlorid wurde in 8 ml trockenem Pyridin gelöst und zu einem Gemisch von 2,15 g [1S-[1α(4R*,6R*),2α,6β,8β,8aα]]-4-[[(1,1-Dimethyläthyl)dimethylsilyl]oxy[2-(1,2,6,7,8,8a-hexahydro-8-hydroxy-2,6-dimethyl-1-naphthalinyl)äthyl]-tetrahydro-2H-pyran-2-on, 0,35 g 4-Pyrrolidinopyridin und 10 ml trockenem Pyridin gegeben. Das Gemisch wurde über Nacht auf 50° erwärmt, dann gekühlt und mit Eisstücken versetzt. Das Gemisch wurde in Diäthyläther aufgenommen und nacheinander mit Wasser, verdünnter Schwefelsäure und gesättigter Natriumbicarbonatlösung gewaschen. Das Gemisch wurde über Natriumsulfat getrocknet, filtriert und unter vermindertem Druck konzentriert. Der Rückstand wurde an Silicagel mit Hexan/Dichlormethan/Aethylacetat (25:25:50) chromatographiert und lieferte 0,314 g 3,3-Difluor-2,2-dimethylbutansäure [1S-[1α,3α,7β,8β-(2R*,4R*)8aβ]]-8-[2[Tetrahydro-4-[[(1,1-dimethyläthyl]dimethylsilyl]oxy]-6-oxo-2H-pyran-2-yl]äthyl]-1,2,3,7,8,8ahexahydro-3,7-dimethyl-1-naphthalinylester.

## Beispiel 3

Ein Gemisch von 0,46 g 3,3-Difluor -2,2-dimethylbutansäure [1S-[1α,3α,7β,8β(2R*,4R*)8aβ]]8-[2-

[Tetrahydro-4-[[(1,1-dimethyläthyl]dimethylsilyl]oxy]-6- oxo-2H-pyran--2-yl]äthyl]-1,2,3,7,8,8a-hexahydro-3,7-dimethyl1-naphthalinylester, 2 ml Tetrahydrofuran, 0,463 ml Essigsäure, 0,103 ml Wasser und 1,85 ml 1M Tetrabutylammoniumfluorid wurde 32 Stunden bei Raumtemperatur gerührt. Das Gemisch wurde in Diäthyläther aufgenommen, der Aetherextrakt nacheinander mit Wasser und Kochsalzlösung gewaschen und dann über Natriumsulfat getrocknet. Das Gemisch wurde abfiltriert und unter vermindertem Druck eingedampft. Chromatographie des Rückstandes an Silicagel mit Hexan/Aethylacetat (1:1) lieferte 0,329 g 3,3-Difluor-2,2-dimethylbutansäure [1S-[1α,3α,7β,8β-(2R*,4R*)8aβ]]-8-[2-[tetrahydro -4-hydroxy-6-oxo-2H-pyran-2-yl]äthyl]-1,2,3,7,8,8a-hexahydro -3,7-dimethyl-1-naphthalinylester.

Ein Analysenpräparat wurde aus Chlorbutan umkristallisiert, Schmelzpunkt 154-159°.

## Beispiel 4

Ein Gemisch von 12,25 g 2,2-Dimethyl-3-oxobutansäureäthylester und 25 g Diäthylaminoschwefeltrifluorid wurde 55 Stunden erwärmt und dann gekühlt. Danach wurde Dichlormethan zugesetzt und das Gemisch auf Eis gegossen. Die Dichlormethanschicht wurde nacheinander mit Wasser, gesättigter Natriumbicarbonatlösung und Kochsalzlösung gewaschen und dann über Natriumsulfat getrocknet. Das Gemisch wurde abfiltriert und eingedampft und lieferte 24,6 g eines Gemisches, das 3,3-Difluor-2,2-dimethylbutansäureäthylester enthielt. Dieses Material wurde 3 3/4 Stunden mit 304 ml 1N Natronlauge zum Rückfluss erhitzt und dann gekühlt. Das Gemisch wurde mit Dichlormethan extrahiert. Der Extrakt wurde mit Wasser gewaschen, über Natriumsulfat getrocknet, filtriert und eingedampft. Der Rückstand wurde 3 3/4 Stunden mit 81 ml 1N Natronlauge zum Rückfluss erhitzt, gekühlt und mit Dichlormethan extrahiert. Die vereinigten wässrigen Phasen wurden angesäuert, auf etwa pH 2 mit verdünnter Schwefelsäure und mit Dichlormethan extrahiert. Dieser Extrakt wurde mit Kochsalzlösung gewaschen, über Natriumsulfat getrocknet, filtriert und eingedampft. Der Rückstand wurde weiter gereinigt, durch Destillation bei 35-40 Torr in einem Eisbad bei 90-120° und lieferte 6 g festes 3,3-Difluor-2,2-dimethylbutansäure. Das Produkt wurde aus Dichlormethan/Pentan umkristallisiert und lieferte 3 g 3,3-Difluor-2,2-dimethylbutansäure, NMR ($CDCl_3$ 200 mHz) 1,73 (t, J=18Hz, 3H), 1,38 (S, 3H).

## Beispiel 5

Eine Formulierung für die orale Verabreichung kann folgende Zusammensetzung haben

| | |
|---|---|
| 1. Verbindung der Formel I or II | 20 Milligram |
| 2. Lactose | 150 Milligram |
| 3. Stärke 1500 | 30 Milligram |
| 4. Talk | 20 Milligram |

Bestandteil 1 wird mit einem Teil 2 gemischt, danach werden 3 und 4 zugesetzt und gemischt. Schliesslich wird der Rest des Bestandteils 2 zugesetzt, gründlich gemischt und die Masse durch eine geeignete Mühle gegeben. Das so erhaltene Präparat wird in Kapseln abgefüllt.

## Beispiel 6

Eine Formulierung für die topische Verabreichung der Verbindungen der Formel I und II kann folgende Zusammensetzung aufweisen

| | |
|---|---|
| 1. Verbindung der Formel I oder II | 10,0 Mikrogramm |
| 2. Stearylalkohol | 4,0 g |
| 3. Cetylalkohol | 4,0 g |
| 4. Mineralöl | 3,0 g |
| 5. Polysorbat 60 | 4,5 g |
| 6. Sorbitanstearat | 4,5 g |
| 7. Propylenglycol | 10,0 g |
| 8. Methylparaben | 0,18 g |
| 9. Propylparaben | |
| 10. Wasser q.s. ad | 100,00 g |

Bestandteile 2-6 werden auf 80° erwärmt bis alles geschmolzen ist. Danach wird Bestandteil 1 in der öligen Phase gelöst. Bestandteil 7 und 10 werden auf 90° erwärmt und die Bestandteile 8 und 9 werden in der so erhaltenen wässrigen Phase gelöst. Danach wird die wässrige Phase zur Oelphase gegeben und rasch gerührt, so dass eine Emulsion erhalten wird. Danach wird langsam auf 50° abkühlen gelassen um die Emulsion zu verfestigen. Unter weiterem Rühren wird das Präparat auf Raumtemperatur abgekühlt.

## Ansprüche

1. Verbindungen der Formel

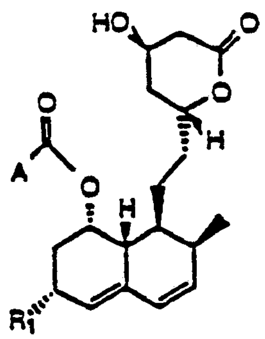

I

und

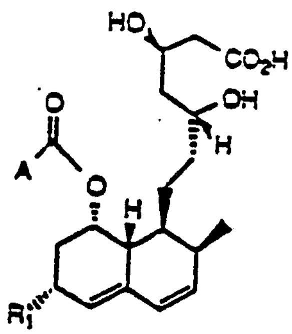

II

worin $R_1$ Wasserstoff oder Methyl, A $R_2$-C(F)$_2$-C($CH_3$)$_2$- oder $R_3$O-C($CH_3$)($R_4$)-ist, wobei $R_2$ Niederalkyl, $R_3$ Phenyl, oder Phenyl, das durch 1-3 Substituenten aus der Gruppe Halogen, Niederakyl und Niederalkoxy substituiert ist, und $R_4$ Wasserstoff oder Methyl darstellt,
und pharmazeutisch anwendbare Salze, $C_{1-4}$-Alkylester, Acetylamino-$C_{1-4}$-alkylester, Phenyl-$C_{1-4}$-alkylester, Dimethylamino-$C_{1-4}$-alkylester und α-Monoglyceride der Verbindungen der Formel II.

2. 3,3-Difluoro-2,2-dimethylbutansäure [1S-[1α,3α,7β,8β(2R*,4R*)8aβ]]-8-[2[tetrahydro-4-hydroxy-6-oxo-2H-pyran-2-yl]äthyl]-1,2,3,7,8,8a-hexahydro-3,7-dimethyl-1-naphthalinylester.

3. 2-(4-Chlorophenoxy)-2-methylpropionsäure [1S-[1α,3α,7β,8β-(2R*,4R*),8aβ]]-1,2,3,7,8,8a-hexahydro-3,7-dimethyl-8-[2-[tetrahydro-4-hydroxy-6-oxo-2H-pyran-2-yl]äthyl]-1-naphthalinylester.

4. Verbindungen der Formel
$R_2$-C(F)$_2$-C($CH_3$)$_2$C(O)X'
worin $R_2$ Niederalkyl und X' Hydroxy, Chlor oder Brom ist.

5. Die Verbindungen der Ansprüche 1-3 zur Verwendung als Heilmittel.

6. Verfahren zur Herstellung der Verbindungen der Ansprüche 1-3, dadurch gekennzeichnet, dass man in einer Verbindung der Formel

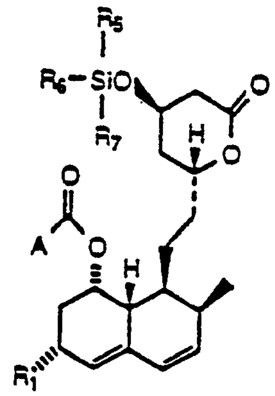

I'

worin A und $R_1$ die in Anspruch 1 angegebene Bedeutung haben und $R_5$, $R_6$ und $R_7$ unabhängig voneinander Niederalkyl oder Phenyl darstellen und nicht mehr als zwei Reste $R_5$, $R_6$ und $R_7$ Phenyl sind, die Silyläthergruppe spaltet und gewünschtenfalls eine enthaltene Verbindung der Formel I in eine Verbindung der Forml II oder einen $C_{1-4}$-Alkylester, Acetylamino-$C_{1-4}$-alkylester, Phenyl-$C_{1-4}$-alkylester, Dimethylamino-$C_{1-4}$-alkylester oder ein α-Monoglycerid der Verbindungen der Formel II umwandelt.

7. Pharmazeutische Präparate, enthaltend eine Verbindung der Formel I oder II gemäss Anspruch 1 oder ein pharmazeutisch anwendbares Salz, einen $C_{1-4}$-Alkylester, Acetylamino-$C_{1-4}$-alkylester, Phenyl-$C_{1-4}$-alkylester, Dimethylamino-$C_{1-4}$-alkylester oder α-Monoglycerid der Verbindung der Formel II.

8. Die Verwendung einer Verbindung der Formel I oder II gemäss Anspruch 1 oder eines pharmazeutisch anwendbaren Salzes, eines $C_{1-4}$-Alkylester, Acetylamino-$C_{1-4}$-alkylester, Phenyl-$C_{1-4}$-alkylester, Dimethylamino-$C_{1-4}$-alkylester oder α-Monoglycerids der Verbindung der Formel II zur Herstellung von Heilmitteln zur Behandlung hyperproliferativer Hauterkrankungen oder Hypercholesterinämie.

Europäisches Patentamt

# EUROPÄISCHER RECHERCHENBERICHT

Nummer der Anmeldung

EP 89 12 0560

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 245 004 (MERCK)<br>* Ansprüche *<br>----- | 1,5 | C 07 D 309/30<br>C 07 C 69/732<br>C 07 C 53/21 |

| RECHERCHIERTE SACHGEBIETE (Int. Cl.5) |
|---|
| C 07 D 309/00<br>C 07 C 69/00<br>C 07 C 59/00<br>C 07 C 53/00 |

Der vorliegende Recherchenbericht wurde für alle Patentansprüche erstellt

| Recherchenort | Abschlußdatum der Recherche | Prüfer |
|---|---|---|
| DEN HAAG | 10-02-1990 | FRANCOIS J.C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)